# EUROPEAN PATENT SPECIFICATION

(11) **EP 2 957 276 B1**
(45) Date of publication and mention of the grant of the patent: **18.12.2019**
(21) Application number: 14752176.9
(22) Date of filing: 04.02.2014
(51) Int. Cl.: A61K 8/365, A61K 8/19, A61K 8/22, A61K 8/38, A61Q 11/00

(54) **DENTAL BLEACHING COMPOSITION**
ZAHNBLEICHUNGSZUSAMMENSETZUNG
COMPOSITION DE BLANCHIMENT DENTAIRE

(30) Priority: 18.02.2013 KR 20130016988
(43) Date of publication of application: 23.12.2015
(73) Proprietor: Osstem Implant Co., Ltd., Seoul 153-759 (KR)
(72) Inventor: SHIN, Seung Hwa, Busan 611-839 (KR); CHUNG, Yong Il, Busan 616-735 (KR); CHOI, Da Mi, Busan 611-830 (KR); KIM, Min Kyoung, Busan 611-808 (KR); SONG, Ju Dong, Busan 609-805 (KR); EOM, Tae Gwan, Busan 612-775 (KR); CHOI, Kyoo Ok, Seoul 153-759 (KR)
(74) Representative: Caspary, Karsten
(86) International application number: PCT/KR2014/000937
(87) International publication number: WO 2014/126351

(56) References cited:
- KR-A- 20040 095 898
- KR-B1- 100 550 065
- US-A1- 2002 076 384
- US-A1- 2003 152 528
- US-A1- 2004 241 110
- US-A1- 2006 099 153
- US-A1- 2007 065 376
- US-A1- 2009 010 859
- US-A1- 2012 322 024

## Description

### TECHNICAL FIELD

The present invention relates to a dental bleaching composition, and more particularly to a dental bleaching composition comprising a metal tartrate chelate complex.

### BACKGROUND ART

In recent years, with a gradual increase in the public expectations for aesthetic needs and appearance, the interest in the tooth whitening field has increased in the dental care. Accordingly, the trend of the dental care has moved from the function-centered dental treatment to the aesthetic dental treatment.

The factors that affect the aesthetic impression of teeth are tooth arrangement, size, color, texture, light permeability, and so forth. Among these, the tooth color is the most sensitive and critical factor, so the yellow discolored teeth possibly cause psychological problems as well as aesthetic problems.

Tooth whitening refers to a procedure on the stained or discolored teeth to restore the natural tooth color using the pharmacological actions of the dental bleaching agent.

In the tooth whitening, the exogenous stains on the surface of teeth caused by the compound such as tannin or polyphenol can be eliminated by removing the staining substances through the physical method such as tooth cleaning. But, the endogenous stains caused by the penetration of the staining substances onto the enamel or the underlying dentin are hard to eliminate by the physical method alone and can be removed by the chemical method eliminating the staining substances with a dental bleaching agent penetrating onto the surface of the teeth in addition to the physical method.

The conventional tooth whitening is performed using hydrogen peroxide or its precursor, e.g., carbamide peroxide.

Hydrogen peroxide generates free radicals that are chemically unstable and have affinity to electrons, so the free radicals bind with other organic molecules to get the chemical stability and simultaneously generate free radicals to achieve tooth whitening. In addition, the free radicals are easy to penetrate onto the surface of teeth, easily moving towards the surface of the teeth and reaching up to the organic substances sticking to the surface of the enamel to oxidize the pigment molecules into tiny colorless particles.

The factors that affect the decomposition of hydrogen peroxide include temperature, pH, metal ions, light, and are known to promote the decomposition of the hydrogen peroxide.

However, besides these factors that promote the decomposition of the hydrogen peroxide, the abilities to maintain the pH value and penetrate the hydrogen peroxide onto the surface of teeth have a great effect on the tooth whitening performance and recently have been considered as significant factors in the development of the dental bleaching composition.

Accordingly, there is a demand for developing a dental bleaching composition that is capable of enhancing the penetrating rate of the conventional dental bleaching composition onto the surface of teeth and maintaining the tooth whitening effect in the long term.

US 2002/0076384 A1 discloses teeth whitening chewing gum.

US 2004/0241110 A1 discloses jelly-type tooth-bleaching patches.

US 2012/0322024 A1 discloses a strip for application to teeth.

US 2009/0010859 A1 discloses clear gel toothpaste.

### DISCLOSURE

### TECHNICAL PROBLEM

One embodiment of the present invention is to provide a dental bleaching composition comprising a metal tartrate chelate complex.

### TECHNICAL SOLUTION

In accordance with one aspect of the present invention, there is provided a dental bleaching composition according to claim 1 including a dental bleaching agent, a binder, a metal tartrate chelate complex, and a solvent.

The dental bleaching agent includes **peroxide, hypochlorite, percarbonate, peroxyacid,** or a combination thereof.

The binder includes a polymer.

The polymer has a number averaged molecular weight of 500,000 to 4,000,000.

The tartrate includes monosodium tartrate, disodium tartrate, sodium ammonium tartrate, monopotassium tartrate, dipotassium tartrate, potassium sodium tartrate, calcium tartrate, or a combination thereof.

The solvent includes water, ethanol, or a combination thereof.

The dental bleaching composition includes 100 parts by weight of the dental bleaching agent, 30 to 1,700 parts by weight of the binder, 0.3 to 350 parts by weight of the tartrate, and 300 to 3,200 parts by weight of the solvent.

The dental bleaching composition includes a metal component.

The metal component is derived from a metal salt.

The metal salt includes iron salt, copper salt, or a combination thereof.

The dental bleaching composition may further include a pH regulator.

The pH regulator may include sodium hydroxide, sodium carbonate, sodium bicarbonate, potassium carbonate, tris(hydroxymethyl)aminomethane, triethanol amine, or a combination thereof.

The content of the dental bleaching agent may be 3 to 35 parts by weight with respect to 100 parts by weight of the dental bleaching composition.

### ADVANTAGEOUS EFFECT

The dental bleaching composition according to one embodiment of the present invention contains tartrate of proven stability as an additive for food, so it has no harm to the human body and works well to prevent the reduction of metal ions present on the surface of the teeth and the consequent deactivation of the metal ions caused by the reduction and the built-up of the metal formed by the deactivation of the metal ions, thereby to maintain the pH value of the tooth surface high and enhance the penetration of the dental bleaching agent onto the surface of the teeth and the decomposition rate of the dental bleaching agent,resulting in improving the dental bleaching performance.

### BRIEF DESCRIPTION OF THE DRAWINGS

FIG. 1 is an image showing the dental bleaching effect of the dental bleaching composition according to one embodiment of the present invention.
FIG. 2 is a graph showing the change in the lightness of teeth as a function of the frequency of bleaching when using the dental bleaching composition according to one embodiment of the present invention.

### PREFERRED EMBODIMENTS OF THE INVENTION

Hereinafter, a detailed description will be given as to a dental bleaching composition according to one embodiment of the present invention.

The dental bleaching is according to claim 1 and includes a dental bleaching agent, a binder, a metal tartrate chelate complex, and a solvent.

The dental bleaching agent, when breaks down, reacts with the stained components (e.g., proteins) present on the surface of the teeth to eliminate the stained components.

The dental bleaching agent includes **peroxide, hypochlorite, percarbonate, peroxyacid, or a combination thereof.**

The peroxide includes hydrogen peroxide, sodium peroxide, potassium peroxide, carbamide peroxide, sodium perborate, sodium percarbonate, or a combination thereof.

The hypochlorite may include sodium hypochlorite, potassium hypochlorite, calcium hypochlorite, barium hypochlorite, or a combination thereof.

The percarbonate may include sodium percarbonate, potassium percarbonate, or a combination thereof.

The peroxyacid may include peroxyacetic acid, peroxy monophosphoric acid, peroxy diphosphoric acid, peroxy monosulfuric acid, peroxy disulfuric acid, peroxy nitric acid, or a combination thereof.

The binder functions to bind the constituent ingredients of the dental bleaching composition. Accordingly, the dental bleaching composition may form a gel.

The binder includes a polymer having a number averaged molecular weight of 500,000 to 4,000,000. For example, the binder may include an ionic polymer, a nonionic polymer, or a combination thereof.

The ionic polymer may include carboxymethyl cellulose, carboxypropyl cellulose, or a combination thereof.

The nonionic polymer may include polyvinyl alcohol, polyvinylpyrrolidone, hydroxyethyl cellulose, hydroxypropyl cellulose, polyacrylic acid, or a combination thereof.

The tartrate is to prevent the reduction of the metal ion present on the surface of teeth (e.g., the reaction between the metal ion and the hydroxyl group on the surface of the teeth) and the consequent deactivation of the metal ion (i.e., the decrease in the ability of decomposing the dental bleach) and the build-up of the metal formed by the deactivation of the metal ion. In other words, the tartrate is used as a chelating agent that can form coordinate bonds to the metal ion to make a chemically stable chelate complex.

In addition, the tartrate can prevent the deactivation of the metal ion and maintain the high level of the decomposition rate of the dental bleaching agent promoted by the metal ion to maintain the high concentration of the hydroxyl group generated by the decomposition of the dental bleaching agent and increase the pH value on the surface of the teeth, maintaining the surface of the teeth alkaline and thereby enhancing the tooth whitening effect. The tartrate also prevents the build-up of the metal produced by the reduction of the metal ion (that is, the deposit of the metal on the surface of the teeth), making it easier for the dental bleaching agent to penetrate onto the surface of the teeth and thus maintaining the decomposition rate of the dental bleaching agent high, to enhance the tooth whitening effect.

The tartrate is an additive for food that is of proven stability because it is very soluble to an aqueous solution irrespective of the pH value, hence easy to formulate and harmless to the human body.

The tartrate includes monosodium tartrate, disodium tartrate, sodium ammonium tartrate, monopotassium tartrate, dipotassium tartrate, potassium sodium tartrate, calcium tartrate, or a combination thereof.

The solvent functions to dissolve at least the dental bleaching agent and the binder. The solvent includes water, ethanol, or a combination thereof.

The dental bleaching composition includes 100 parts by weight of the dental bleaching agent, 30 to 1,700 parts by weight of the binder, 0.3 to 350 parts by weight of the tartrate, and 300 to 3,200 parts by weight of the solvent.

When the contents of the binder and the solvent are within the above-defined ranges with respect to 100 parts by weight of the dental bleaching agent, the viscosity of the dental bleaching composition has such an appropriate value as not to make the dental bleaching composition easily diluted or lost by the saliva and helps apply the dental bleaching composition to the teeth, thereby providing good phase stability.

When the content of the tartrate is within the defined range with respect to 100 parts by weight of the dental bleaching agent, it enhances the phase stability of the dental bleaching agent in the dental bleaching composition and provides good tooth whitening effect.

The dental bleaching composition further includes a metal component.

The metal component has affinity to the constituent ingredients (e.g., inorganic substances) of the enamel and tends to move towards the enamel when the dental bleaching composition is applied on the surface of the teeth. Accordingly, the metal component (particularly, metal ions) decomposes the dental bleaching agent on the surface of the teeth or its vicinity to help the effective removal of the stained components deep inside the enamel, thereby increasing the tooth whitening effect. In this manner, the metal component promotes the decomposition of the dental bleaching agent to activate the tooth whitening and enables a fast and powerful tooth whitening effect.

The metal component is derived from a metal salt. Such a metal salt may include a metal ion, a central metal forming a chelate compound, or a combination thereof.

The metal salt includes iron salt, copper salt, or a combination thereof.

The iron salt may include at least one compound selected from the group consisting of iron (II) chloride, iron (III) chloride, iron bromide, iron sulfate, iron nitrate, iron (II) acetate, iron propionate, iron citrate, iron gluconate, iron lactate, iron oxide, iron hydroxide, a hydrate thereof, and a ferrous or a ferric compound of a combination thereof.

The copper salt may include copper pyrophosphate, copper sulfate, copper nitrate, copper (II) acetate, copper (I) chloride, copper (II) chloride, copper carbonate (CuCO₃), copper gluconate, copper hydroxide, or a combination thereof.

The concentration of the metal ion in the dental bleaching composition ranges from 0.05 mM to 1 mM. The dental bleaching composition includes a chelate compound (i.e., a complex in which tartrate is bonded to a metal ion), so the central metal of the chelate compound is incorporated into the calculation of the concentration of the metal ion. When the concentration of the metal ion is within the defined range, it can provide a good tooth whitening effect without additional discoloration caused by an excess of the metal ion.

The dental bleaching composition may further include a pH regulator.

The pH regulator is to promote the decomposition of the dental bleaching agent. The pH regulator may include sodium hydroxide, sodium carbonate, sodium bicarbonate, potassium carbonate, tris(hydroxymethyl)aminomethane, triethanol amine, or a combination thereof.

The content of the pH regulator may be 1 to 20 parts by weight with respect to 100 parts by weight of the dental bleaching agent. When the content of the pH regulator is within the defined range with respect to 100 parts by weight of the dental bleaching agent, the pH value of the dental bleaching composition becomes optimal to eliminate the risk of stimulating oral mucous membranes and leads to sufficient decomposition of the dental bleaching agent, thereby providing a good tooth whitening effect.

The dental bleaching composition may further include other additives, such as a flavorant, a desensitizer, an anti-cavity agent, an anti-gingivitis agent, or a combination thereof.

The flavorant may include peppermint, spearmint, citrus, herb, menthol, or a combination thereof.

The desensitizer may include potassium nitrate, stannous fluoride, sodium monofluoride phosphate (MFP), sodium fluoride (NaF), sodium silicofluoride (Na₂SiF₆), or a combination thereof.

The anti-cavity agent may include sodium fluoride (NaF), calcium fluoride (CaF₂), or a combination thereof.

The anti-gingivitis agent may include bamboo salt, tocopherol acetate, vitamin D, alantoin chlorohydroxy aluminum, triclosan, xylitol, calcium glycerol phosphate, aminocaproic acid, KNO₃, sodium monofluorophosphate, enzyme, or a combination thereof.

The content of the other additives may be appropriately controlled within such a range that it can improve the after-use feeling and functionality of the dental bleaching composition without a significant change in the form and stability of the dental bleaching composition.

The content of the dental bleaching agent may be 3 to 35 parts by weight with respect to 100 parts by weight of the dental bleaching composition. When the content of the dental bleaching agent is within the defined range with respect to 100 parts by weight of the dental bleaching composition, it can provide a good tooth whitening effect without a risk of stimulating gum and oral mucous membranes.

Hereinafter, the present invention will be described in further detail with reference to examples, which are not intended to limit the scope of the present invention.

### [Examples]

### Reference Example 1: Preparation of tartrate-containing dental bleaching composition

100 parts by weight of hydrogen peroxide, 166.7 parts by weight of polyacrylic acid (Sigma Aldrich, Carbomer, weight averaged molecular weight: 3,000,000), 33.3 parts by weight of monosodium tartrate (Yakuri Chemicals), and 3,033.3 parts by weight of distilled water are mixed together to prepare a dental bleaching composition.

### Comparative Example 1: Preparation of dental bleaching composition not containing chelating agent

100 parts by weight of hydrogen peroxide, 166.7 parts by weight of polyacrylic acid (Sigma Aldrich, Carbomer, weight averaged molecular weight: 3,000,000), and 3,000 parts by weight of distilled water are mixed together to prepare a dental bleaching composition.

### Comparative Example 2: Preparation of EDTA (Ethylene Diamine Tetra-Acetate)-containing dental bleaching composition

100 parts by weight of hydrogen peroxide, 166.7 parts by weight of polyacrylic acid (Sigma Aldrich, Carbomer, weight averaged molecular weight: 3,000,000), 33.3 parts by weight of EDTA (Sigma Aldrich), and 3,033.3 parts by weight of distilled water are mixed together to prepare a dental bleaching composition.

### [Assessment Examples]

### Assessment Example 1: Tooth whitening effect

The dental bleaching composition prepared in Reference Example 1 is applied to four human teeth that have the similar shade stage and the bleached teeth are visually observed to evaluate the tooth whitening effect according to the VITA Shape Classical Guide. More specifically, the application of the dental bleaching composition is carried out in the following procedures.

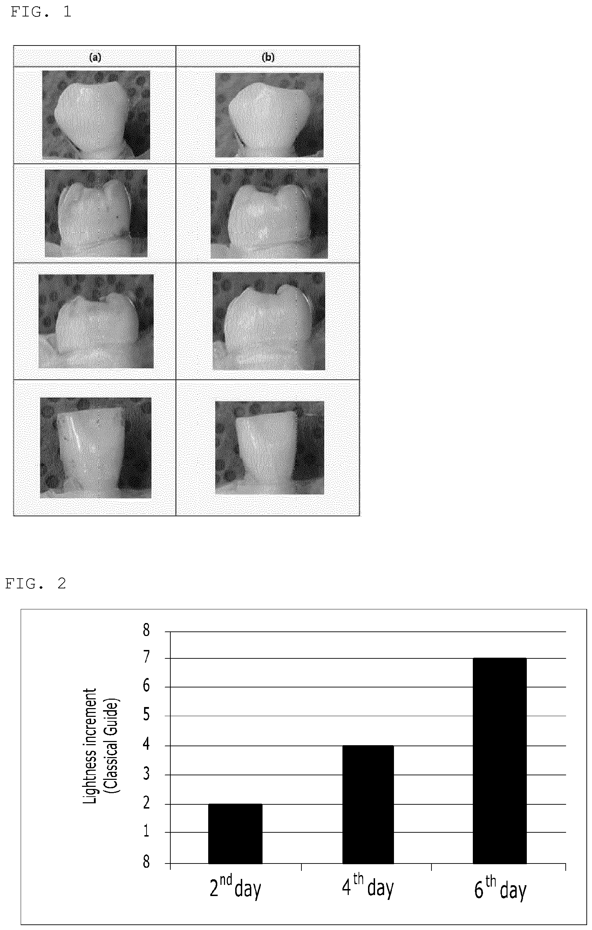

In other words, the dental bleaching composition is applied to the individual teeth with a brush twice a day for 6 days. The photographs of the teeth are taken before and after the six-day bleaching treatment and presented in FIG. 1. The tooth whitening effect on the teeth is evaluated on the second day, the fourth day and the sixth day of the bleaching treatment. For the four bleaches teeth, the average of the lightness increment (that is, the shade stage increment) in contrast to the untreated teeth is calculated. The calculation results are presented in FIG. 2.

In FIG. 1, the teeth in column (a) are those before the bleaching treatment, and the teeth in column (b) are those after the bleaching treatment.

Referring to FIGS. 1 and 2, even though the bleaching treatment is carried out for a short period of time within six days, the teeth are improved in whiteness by six to seven shade stages in contrast to the teeth before the bleaching treatment. In addition, it is revealed that the lightness (shade stage) increment of the bleached teeth is increased with an increase in the number of days under bleaching treatment.

### Assessment 2: Color change of teeth

The dental bleaching compositions prepared in Reference Example 1 and Comparative Examples 1 and 2 are used to bleach the three human teeth of the similar shade stage twice a day for 5 days. In contrast to the untreated teeth, the teeth bleached for one day, two days, three days, four days, and five days are measured in regards to the color change (ΔE*ab). The results are presented in Table 1. During the bleaching treatment period, the individual dental bleaching compositions are stored at 37 °C. The numeral values indicated in Table 1 are the color change (ΔE*ab). Here, a spectrophotometer (Spectro Color Meter SE-2000, Nippon Denshoku Industries Co., Ltd., Tokyo, Japan) is used as the measurement instrument of the color change (ΔE*ab).

**[Table 1]**

| Number of days under bleaching treatment | Comparative Example 1 | Comparative Example 2 | Example 1 |
|---|---|---|---|
| 1 day | 1.5 ± 0.7 | 1.8 ± 1.5 | 4.2 ± 1.6 |
| 2 days | 1.7 ± 0.5 | 2.0 ± 1.8 | 4.5 ± 2.0 |
| 3 days | 1.8 ± 0.8 | 2.2 ± 2.6 | 4.9 ± 2.6 |
| 4 days | 2.0 ± 1.1 | 2.8 ± 2.8 | 5.8 ± 2.8 |
| 5 days | 2.9 ± 1.1 | 3.3 ± 3.5 | 6.2 ± 3.5 |

Referring to Table 1, it is revealed that the dental bleaching composition prepared in Reference Example 1 is superior in the tooth whitening effect to the dental bleaching compositions prepared in Comparative Examples 1 and 2.

Although a few exemplary embodiments of the present invention have been shown and described, the present invention is not limited to the described exemplary embodiments.

## Claims

1. A dental bleaching composition comprising a dental bleaching agent, a binder, tartrate, and a solvent;
wherein the dental bleaching agent comprises peroxide, hypochlorite, percarbonate, peroxyacid, or a combination thereof;
wherein the binder comprises a polymer, the polymer having a number averaged molecular weight of 500,000 to 4,000,000; wherein the tartrate comprises monosodium tartrate, disodium tartrate, sodium ammonium tartrate, monopotassium tartrate, dipotassium tartrate, potassium sodium tartrate, calcium tartrate, or a combination thereof;
wherein the solvent comprises water, ethanol, or a combination thereof;
wherein the dental bleaching composition comprises 100 parts by weight of the dental bleaching agent, 30 to 1,700 parts by weight of the binder, 0.3 to 350 parts by weight of the tartrate, and 300 to 3,200 parts by weight of the solvent;
wherein the dental bleaching composition further comprises a metal component, wherein the metal component is derived from a metal salt;
wherein the metal salt comprises iron salt, copper salt, or a combination thereof;
wherein the concentration of the metal ions of metal salt within the dental bleaching agent composition ranges from 0.05 mM to 1 mM; and wherein said tartrate acts as a chelating agent that forms coordinate bonds to the metal ions to make a chemically stable chelate complex.

2. The dental bleaching composition as claimed in claim 1, wherein the composition additionally comprises a pH regulator comprising sodium hydroxide, sodium carbonate, sodium bicarbonate, potassium carbonate, tris(hydroxymethyl)aminomethane, triethanol amine, or a combination thereof.

3. The dental bleaching composition as claimed in claim 1, wherein the content of the dental bleaching agent is 3 to 35 parts by weight with respect to 100 parts by weight of the dental bleaching composition.

## Patentansprüche

1. Zahnbleichzusammensetzung mit einem Zahnbleichmittel, einem Bindemittel, einem Tartrat, und einem Lösungsmittel;
wobei das Zahnbleichmittel Peroxid, Hypochlorit, Percarbonat, Persäure, oder eine Kombination davon aufweist;
wobei das Bindemittel ein Polymer aufweist, wobei das Polymer ein Zahlengemitteltes Molekulargewicht von 500.000 bis 4.000.000 hat;
wobei das Tartrat Mononatriumtartrat, Dinatriumtartrat, Natriumammoniumtartrat, Monokaliumtartrat, Dikaliumtartrat, Kaliumnatriumtartrat, Kalziumtartrat, oder eine Kombination davon aufweist;
wobei das Lösungsmittel Wasser, Ethanol, oder eine Kombination davon aufweist;
wobei die Zahnbleichzusammensetzung 100 Gewichtsanteile des Zahnbleichmittels, 30 bis 1.700 Gewichtsanteile des Bindemittels, 0,3 bis 350 Gewichtsanteile des Tartrats, und 300 bis 3.200 Gewichtsanteile des Lösungsmittels aufweist;
wobei die Zahnbleichzusammensetzung weiterhin eine Metallkomponente aufweist; wobei die Metallkomponente aus einem Metallsalz stammt;
wobei das Metallsalz Eisensalz, Kupfersalz, oder eine Kombination davon aufweist;
wobei die Konzentration der Metallionen von Metallsalz innerhalb der Zahnbleichmittelzusammensetzung in einem Bereich von 0,05 mM bis 1 mM liegt; und
wobei das Tartrat als ein Chelatmittel fungiert, das koordinative Bindungen der Metallionen bildet, um einen chemisch stabilen Chelatkomplex zu erzeugen.

2. Zahnbleichzusammensetzung nach Anspruch 1, wobei die Zusammensetzung zusätzlich einen pH-Regulator aufweist, der Natriumhydroxid, Natriumcarbonat, Natriumbicarbonat, Kaliumcarbonat, Tris(hydroxymethyl)aminomethan, Triethanolamin, oder eine Kombination davon aufweist.

3. Zahnbleichzusammensetzung nach Anspruch 1, wobei sich der Inhalt des Zahnbleichmittels auf 3 bis 35 Gewichtsteile in Bezug auf 100 Gewichtsteile der Zahnbleichzusammensetzung beläuft.

## Revendications

1. Composition de blanchiment dentaire comprenant un agent de blanchiment dentaire, un liant, du tartrate, et un solvant,
dans laquelle l'agent de blanchiment dentaire comprend du peroxyde, de l'hypochlorite, du percarbonate, du peroxyacide, ou une combinaison de ceux-ci,
dans laquelle le liant comprend un polymère, le polymère ayant un poids moléculaire moyen en nombre de 500 000 à 4 000 000,
dans laquelle le tartrate comprend du tartrate monosodique, du tartrate disodique, du tartrate d'ammonium et de sodium, du tartrate monopotassique, du tartrate dipotassique, tartrate de sodium et de potassium, du tartrate de calcium, ou une combinaison de ceux-ci, dans laquelle le solvant comprend de l'eau, de l'éthanol, ou une combinaison de ceux-ci,
dans laquelle la composition de blanchiment dentaire comprend 100 parties en poids de l'agent de blanchiment dentaire, 30 à 1700 parties en poids du liant, 0,3 à 350 parties en poids du tartrate, et 300 à 3200 parties en poids du solvant,
dans laquelle la composition de blanchiment dentaire comprend en outre un composant métallique, dans laquelle le composant métallique est dérivé d'un sel métallique ; dans laquelle le sel métallique comprend du sel de fer, du sel de cuivre, ou une combinaison de ceux-ci,
dans laquelle la concentration des ions métalliques du sel métallique dans la composition de l'agent de blanchiment dentaire varie de 0,05 mM à 1 mM et
dans laquelle ledit tartrate agit en tant qu'agent chélateur qui forme des liaisons de coordination avec les ions métalliques pour obtenir un complexe chélaté chimiquement stable.

2. Composition de blanchiment dentaire selon la revendication 1, dans laquelle la composition comprend en outre un régulateur de pH comprenant de l'hydroxyde de sodium, du carbonate de sodium, du bicarbonate de sodium, du carbonate de potassium, du tris(hydroxyméthyl)aminométhane, de la triéthanolamine, ou une combinaison de ceux-ci.

3. Composition de blanchiment dentaire selon la revendication 1, dans laquelle la teneur en agent de blanchiment dentaire est de 3 à 35 parties en poids par rapport à 100 parties en poids de la composition de blanchiment dentaire.
